# EUROPEAN PATENT SPECIFICATION

(11) **EP 4 251 231 B1**
(45) Date of publication and mention of the grant of the patent: **18.03.2026**
(21) Application number: 21819023.9
(22) Date of filing: 24.11.2021
(51) Int. Cl.: A61M 3/02

(54) **LIQUID CONTAINER FOR AN ANAL IRRIGATION SYSTEM**
BEHÄLTER FÜR FLÜSSIGKEIT FÜR EIN SYSTEM ZUR ANALEN IRRIGATION
RÉCIPIENT POUR UN LIQUIDE POUR UN SYSTÈME DE'IRRIGATION ANALE

(30) Priority: 24.11.2020 DK PA202070781
(43) Date of publication of application: 04.10.2023
(73) Proprietor: Coloplast A/S, 3050 Humlebaek (DK)
(72) Inventor: ZEUTHEN, Kristoffer, 2100 København Ø (DK); KNOEDLER, Stephanie, 2990 Nivaa (DK); JENSEN, Nina Hoegh, 2200 Copenhagen N (DK)
(86) International application number: PCT/DK2021/050344
(87) International publication number: WO 2022/111778

(56) References cited:
- WO-A1-2019/005836
- US-A1- 2020 206 411
- US-B2- 10 765 796

## Description

The invention relates to a liquid container for an anal irrigation system.

### Brief Description of the Drawing

The accompanying drawings are included to provide a further understanding of embodiments and are incorporated into and a part of this specification. The drawings illustrate embodiments and together with the description serve to explain principles of embodiments. The elements of the drawings are not necessarily to scale relative to each other. Like reference numerals designate corresponding similar parts.
Figure 1 illustrates a perspective view of an anal irrigation system in a bag,
Figure 2 illustrates a perspective view of a bag, a liquid container, and a pump base unit for an anal irrigation system,
Figure 3 illustrates a handle for a liquid container,
Figure 4 illustrates four views, a side view, a front view, a perspective view and a top view, of a liquid container for an anal irrigation system, and
Figure 5 illustrates a schematic view of the detachable liquid container having the rotatable handle in a first position and a second position.

### Background

US2020/206411 relates lo hollow organ irrigation devices, system, and methods. More particularly, the disclosure relates to liquid reservoirs, catheters, and catheter retaining elements that may be employed in hollow organ irrigation devices, systems, and methods. US10765796 B2 discloses a trans anal irrigation device that can be used on a bed.

### Detailed Description

Embodiments, and features of the various exemplary embodiments described in this application, may be combined with each other ("mixed and matched"), unless specifically noted otherwise.

In the following detailed description, reference is made to the accompanying drawings, which form a part hereof, and in which is shown by way of illustration specific embodiments in which the invention may be practiced. Because components of embodiments can be positioned in different orientations, the directional terminology is used for purposes of illustration. The scope of the present invention is defined by the appended claims.

Anal irrigation is one of a number of treatments used to aid people with bowel problems. People suffering from bowel problems are often paralyzed, typically due to spinal cord injuries, and confined to a wheelchair or hospitalized. In these situations, often the peristaltic functions, i.e. the reflexes and muscles of the bowel, cannot be stimulated correctly. This results in constipation or random discharge of bowel contents. By using anal irrigation, a stimulation of the peristaltic movements of the colon can be provided. To perform such anal irrigation, a device comprising an anal probe, also called anal catheter, rectal catheter or speculum, is provided. The anal probe is inserted into the rectum through the anus. The anal probe is typically retained in the rectum by retention means, most commonly a balloon, which is inflated against the wall of the rectum. A liquid, such as water or a saline solution, is then introduced into the rectum through the anal probe. The amount of liquid is generally up to 1.5 liters, depending on the user.

Users of anal irrigation systems typically have poor dexterity. Therefore, simple systems are advantageous for the users.

Embodiments relate to a liquid container for an anal irrigation system, where the liquid container comprises a top part and a bottom wall, at least one side wall connecting the bottom wall with the top part forming a cavity for storage of liquid, where the liquid container comprises a filling spout located in the at least one side wall such that the filling spout is closer to the top part than the bottom part, the filling spout having a periphery, and where the liquid container further comprises a handle, characterized in that the handle is rotatable around the periphery of the filling spout, the liquid container being configured for allowing the user to lift and hold the container in different positions.

The rotatable handle as described above is very convenient to use because the position of the handle allows the user to lift and hold the container in different positions, e.g. in an upright position and/or in a horizontal position. The handle is positioned and rotatable such that the centre of gravity of the liquid container will align the liquid container vertically below the handle in at least two different positions of the handle. Thus, the handle may be arranged in different positions enabling the user to hold/lift the liquid container in a steady and stable manner in various positions.

By rotating the handle to another position, the user may lift the liquid container from an upright storage position and hold the liquid container in a substantially horizontal position, e.g. when filling the liquid container with liquid. Thus, the rotatable handle allows the user to handle the liquid container during preparation of the anal irrigation process. Furthermore, the handle requires a minimum of storage space, e.g. in a bag, when the handle is rotated to a position aligned juxtaposed the front of the liquid container.

In embodiments, the liquid container comprises a spatial oblong shape adapted to allow a user to hold the liquid container by the handle in a substantially vertically or horizontally position.

The liquid container can be disconnected from the anal irrigation system, such that a relatively heavy pump base unit does not have to be lifted together with the liquid container during preparation of the anal irrigation system. The liquid container may be (re-)filled with irrigation liquid, such as water from a tap/faucet in a lavatory.

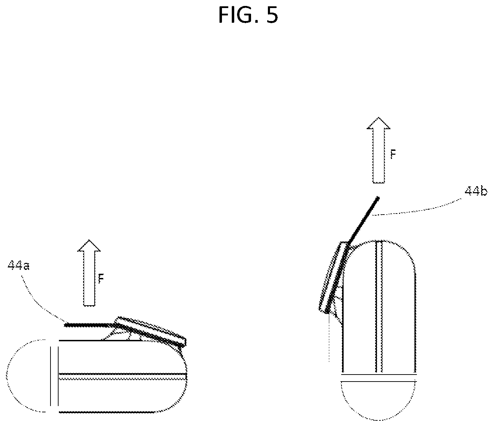

The terms on directional terminology, such as "top," "bottom," "front," "rear," "vertical," "horizontal," etc., are used with reference to the orientation of the liquid container positioned on top of a pump base unit standing on a horizontal surface in an up-right position as the figures illustrate. Thus, the bottom wall extends in a plane substantially parallel to a horizontal surface. Additionally, the terms first direction (x), second direction (y) and third (z) direction are used, all directions extend perpendicular to each other, when the anal irrigation system is in use configuration and the liquid container is connected to the pump base unit (illustrated in figure 5). The first direction (x) extends substantially parallel along the front of the liquid container. The second direction (y) extends perpendicular to the first direction and is substantially equal to an imaginary vertical line of gravity, when the liquid container is in an up-right position. The third direction (z) extends transverse to the first and second direction (x,y) extending in a direction corresponding to the depth of the liquid container.

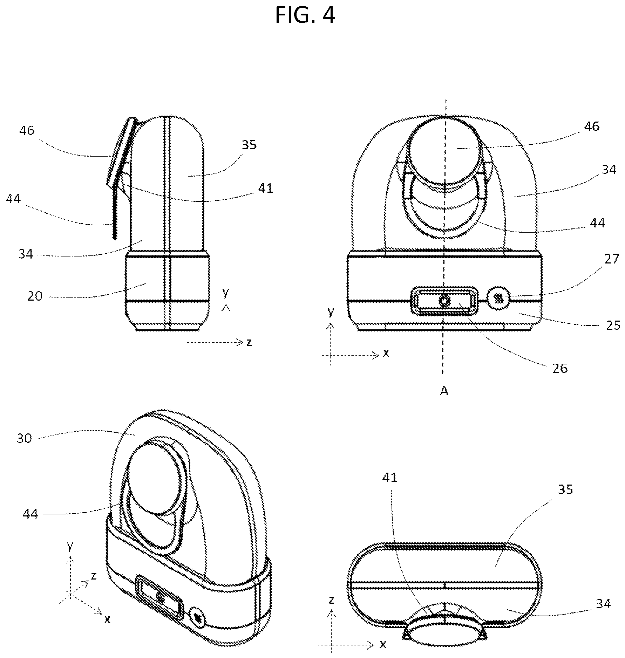

The longitudinal central axis A is defined by a line of symmetry of the liquid container and extends in parallel with the second direction y. The longitudinal central axis A is substantially equal to an imaginary vertical line of gravity when the liquid container is connected to a pump base unit in irrigation configuration in an up-right position. The longitudinal central axis A is illustrated in figure 4.

The use of the phrase "substantially" as a qualifier to certain features or effects in this disclosure is intended to simply mean that any deviations are within tolerances that would normally be expected by the skilled person in the relevant field.

Embodiments relate to a liquid container as described above, wherein the handle is rotatable in a tangential direction around the periphery of the filling spout and in a distance from a center of the filling spout.

In embodiments, the handle comprises at least a first and a second position relative to the liquid container. It is convenient to be able to position the handle in relation to the use during preparation of the anal irrigation system or when the liquid container is in storage configuration. Generally, the handle is freely rotatable in an angle of at least 180 degrees between the first and second position.

In embodiments, the filling spout comprises a circular opening having a size in the range of 3-15 cm, 4-10 cm. The size is convenient as it allows the user to limit spillage during filling of the liquid container and also provides space for the user to clean the liquid container, e.g. with a brush.

In embodiments, the filling spout comprises a rigid ring-shaped collar surrounding the periphery, the handle being rotatably connected to the ring-shaped collar. Embodiments relate to the ring-shaped collar comprising a ratchet mechanism and/or stop flanges limiting the rotation of the handle. The handle may be rotated 180 degrees between the first and second position. The ratchet mechanism may dictate the direction of rotation and/or stabilize the handle in a position by friction. Hereby the user is provided with a sturdier structure which may be easier for the user when handling or holding the liquid container via the handle.

In embodiments, the material of the handle is made of rigid plastic material. In embodiments, the material of the handle is flexible.

In embodiments, the handle comprises a thickness of more than 2 mm. A thickness of more than 2 mm is contemplated to be a convenient size for a user when gripping the handle and carrying the liquid container.

In embodiments, the handle comprises an eight-shaped inner face having two inwardly extending flanges.

In embodiments, the handle comprises two parts, a gripping part and a spout periphery part, which extend in two planes angled in relation to each other, the angle between the two planes is in the range of 0-45 degrees, such as 5-40 degrees, such as 10-30 degrees. Hereby, the gripping part of the handle is vertically aligned with the centre of gravity of the liquid container allowing the user to hold the liquid container in a substantially horizontal or vertical position during preparation of the anal irrigation system.

In embodiments, the filling spout is positioned at the centre of gravity of the liquid container in a first direction and offset from the centre of gravity of the liquid container in a second direction, the first direction and the second direction extend transverse each other. Hereby, the liquid container may take a different orientation depending on the position of the handle, and the handle requires less storage space when the handle is rotated to a position juxtaposed the front of the liquid container.

Generally, the bottom wall may comprise an oval, ellipse, circular, triangle or similar geometric shape.

In embodiments, the bottom wall comprises an oblong shape. In embodiments, the liquid container comprises an oblong shape in the second direction.

In embodiments, the bottom wall comprises an oblong rounded rectangular shape and the filling spout is positioned such that it is vertically aligned with a middle of one of the sides of the rectangular bottom wall. In embodiments, the filling spout is positioned in the at least one sidewall such that the filling spout is placed symmetrically along the centreline of the at least one sidewall.

In embodiments, the bottom wall comprises an outlet connector for fluidly connecting the liquid container to a pump base unit.

Embodiments relate to an anal irrigation system comprising a liquid container according to the preceding claims and a pump base unit.

Embodiments relate to an anal irrigation system as described above, wherein the liquid container is detachable from the pump base unit.

In embodiments, the irrigation system further comprises tubes and an anal probe.

### Detailed Description of the Drawing

Embodiments, and features of the various exemplary embodiments described in this application, may be combined with each other ("mixed and matched"), unless specifically noted otherwise.

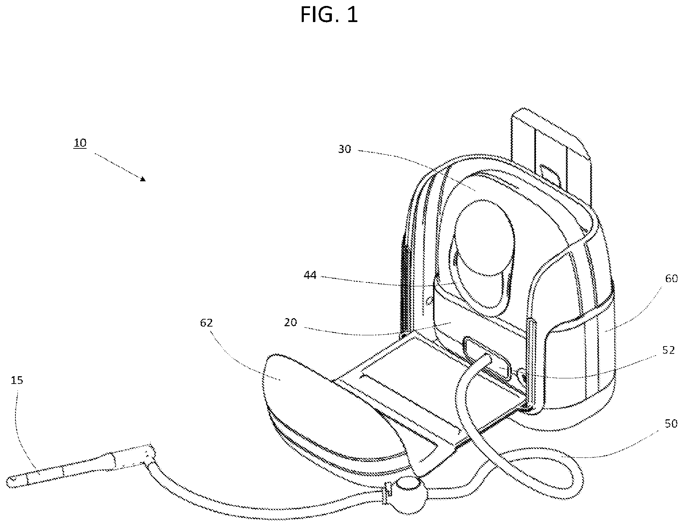

Figure 1 illustrates in a perspective view an anal irrigation system in a use configuration.

The anal irrigation system 10 comprises a pump base unit 20 and a detachable liquid container 30, which is stored in a first cavity of a bag 60. The pump base unit 20 is connected to a probe 15 by a tubing system 50. The tubing system and the probe may be stored in a second cavity of the bag formed by the front wall in front of the pump base unit and the liquid container 30.

The liquid container may be de-attached by grabbing the rotatable handle 44 from the pump base unit. When detached, a user may fill the liquid container with liquid, and then the liquid container may be re-attached to the pump base unit 20 in the bag prior to use of the anal irrigation system.

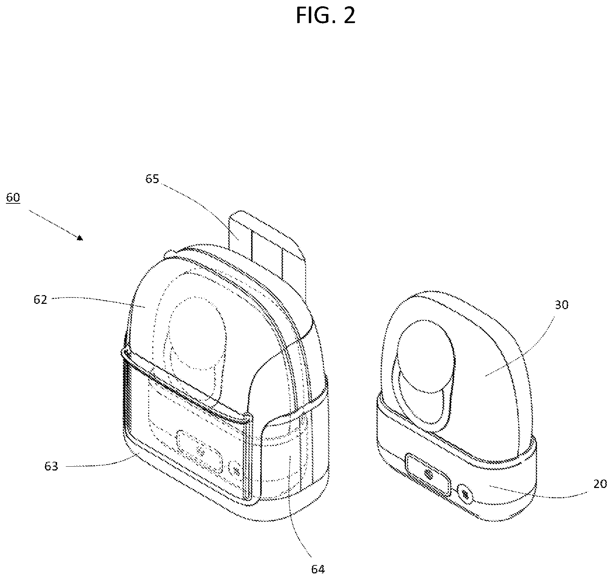

Figure 2 illustrates a perspective view of the detachable liquid container 30 and the pump base unit 20 stored in a closed bag 60. The bag contains the anal irrigation system (illustrated by dotted lines) during storage.

The bag comprises a self-supporting bottom 63 and side walls 64 supporting the pump base unit and the liquid container. The bag may be carried by the handle 65, and when the front wall 62 of the bag is opened, the liquid container and the pump base unit may be removed from the bag.

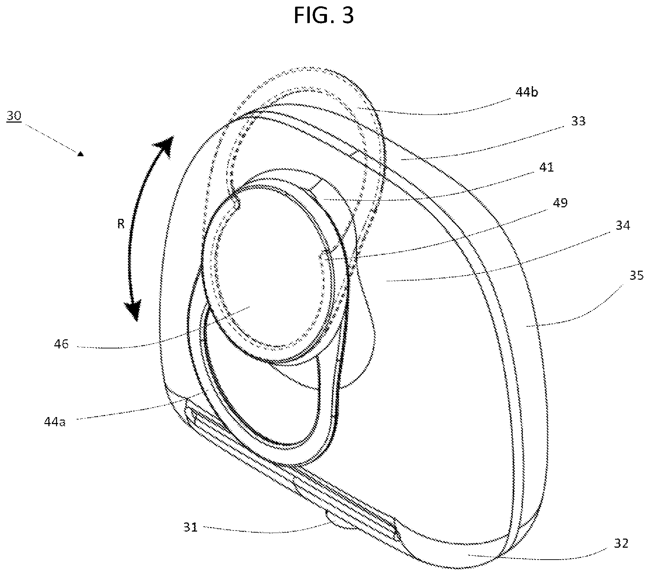

Figure 3 illustrates the rotatable handle 44 for the detachable liquid container 30.

The liquid container comprises a bottom wall 32 having an outlet connector 31 adapted to liquidly connect the container with the pump base unit and the tubing system of an anal irrigation system.

The top part 33 of the liquid container 30 is connected to the bottom wall 32 by the side walls 34, 35. The walls form a cavity for storing liquid. A cross sectional view of the liquid container comprises an oblong rounded rectangular shape having a relatively small depth with respect to the width and the vertical height. The filling spout 41 is arranged at the upper part of the side wall 34 near the top part 33. Thus, the orientation of the liquid container may be changed by the position of the rotatable handle 44.

The rotatable handle is illustrated in a first storage position 44a and by dotted lines in a lifting/carrying position 44b.

A user may rotate the rotatable handle 44 along the arrow R approximately 180 degrees radially around the filling spout 41 from the first position 44a to the second position 44b (illustrated by dotted lines) allowing the user to be able to lift the liquid container e.g. from storage in a bag. By rotating the handle to the first position 44a the centre of gravity for the oblong liquid container changes and the user will be able to hold the liquid container in a substantially horizontal position (also illustrated in figure 5).

The filling spout 41 of the liquid container 30 comprises an opening and a closing cap 46.

The rotatable handle 44 comprise an eight-shaped inner face having two inwardly extending flanges 49. One part of the eight-shaped inner face surrounds the filling spout 41 and the other part of the eight-shaped inner face functioning as a handle part allowing a user to grip the handle.

Figure 4 illustrates four views, respectively a side view, a front view, a perspective view and a top view, of a liquid container and a pump base unit 20 for an anal irrigation system.

The filling spout and the rotatable handle 44 are positioned centred at the longitudinal central axis A of the liquid container at the upper part of the liquid container. The spatial structure of the liquid container has different extends in the first, second and third directions x, y, z.

The liquid container has a front side wall 34 and a rear side wall 35. The extend of the front side wall in the first direction x is larger than the extend of the depth of liquid container in the third direction z. In the third direction the filling spout 41 is positioned offset relative to the centre of gravity of the liquid container.

The front of the liquid container is slightly curved and the periphery of the filling spout extends in a plane not coinciding with any of the three directions x, y, and z. The handle comprises two parts, a gripping part, and a spout periphery part, which extend in two planes angled in relation to each other, the angle between the two planes being in the range of approximately 10-30 degrees. The handle gripping part is adjacent to the front wall 34 and thus compact when stored.

Figure 5 illustrates a schematic view of the liquid container having the rotatable handle in the first position 44a and the second position 44b. In both views, the grip part of the handle is positioned vertically above the centre of gravity. A lifting force F from a user carrying the liquid container by holding the handle is illustrated by an arrow. When the liquid container is lifted by the handle, the centre of gravity of the liquid container will align the liquid container vertically below the handle, thus the handle may be arranged in different positions enabling the user to hold/lift the liquid container having various orientations/positions.

The rotatable handle is illustrated in a first storage position 44a and the centre of gravity for the oblong liquid container allows a user to hold the liquid container via the handle in a substantially horizontal position. By rotating the handle approximately 180 degrees around the filling spout, the handle extends above the top part of the container, and as the handle vertically aligns the centre of gravity of the liquid container, the orientation of the liquid container changes and the new position of the handle allows a user to carry/lift/hold the liquid container in a substantially vertical direction.

The grip part of the handle extends in a plane defining an angle relative to a plane defined by the periphery part of the handle. The small angle between the two parts of the handle may be in the range of 0-45 degrees.

## Claims

1. A liquid container (30) for an anal irrigation system, where the liquid container (30) comprises a top part (33) and a bottom wall, at least one side wall (34) connecting the bottom wall (32) with the top part (33) forming a cavity for storage of liquid, where the liquid container (30) comprises a filling spout (41) located in the at least one side wall (34) such that the filling spout (41) is closer to the top part (33) than the bottom part, the filling spout (41) having a periphery, and where the liquid container (30) further comprises a handle (44), **characterized in that** the handle (44) is rotatable around the periphery of the filling spout (41), the liquid container (30) being configured for allowing the user to lift and hold the container (30) in different positions (44a,44b).

2. The liquid container (30) according to claim 1, wherein the handle (44) is rotatable in a tangential direction around the periphery and in a distance from a center of the filling spout.

3. The liquid container according to any of the preceding claims, wherein the handle (44) comprises at least a first and a second position (44a,44b) relative to the liquid container (30).

4. The liquid container according to claim 3, wherein at least a part of the handle (44) extends beyond the top part (33) of the liquid container, when the handle (44) is in the second position (44b).

5. The liquid container according to any of the preceding claims, wherein the handle (44) comprises an eight-shaped inner face having two inwardly extending flanges (49).

6. The liquid container according to any of the preceding claims, wherein the handle (44) comprises two parts, a gripping part and a spout periphery part, which extend in two planes angled in relation to each other, where the angle between the two planes is in the range of 0-45 degrees, such as 5-40 degrees, such as 10-30 degrees.

7. The liquid container according to any of the preceding claims, wherein the filling spout (41) is positioned at the centre of gravity of the liquid container (30) in a first direction (x) and offset the centre of gravity of the liquid container (30) in a second direction (y), the first direction (x) and the second direction (y) extending transverse each other.

8. The liquid container according to any of the preceding claims, wherein the bottom wall (32) comprises an outlet connector (31) for fluidly connecting the liquid container (30) to a pump base unit (20).

9. The liquid container according to any of the preceding claims, wherein the filling spout (41) is positioned in the at least one side wall (34) such that the filling spout (41) is placed symmetrically along the centreline of the at least one side wall (34).

10. The liquid container according to any of the preceding claims, wherein the bottom wall (32) comprises an oblong rounded rectangular shape and the filling spout (41) is positioned such that it is vertically aligned with a middle of one of the sides of the rectangular bottom wall (32).

11. The liquid container according to any of the preceding claims, wherein the filling spout (41) comprises a rigid ring-shaped collar surrounding the periphery, the handle (44) being rotatably connected to the ring-shaped collar.

12. The liquid container according to any of the preceding claims, wherein the material of the handle (44) is made of rigid plastic material.

13. The liquid container according to any of the preceding claims, wherein the handle (44) comprises a thickness of more than 2 mm.

14. An anal irrigation system comprising a liquid container (30) according to the preceding claims and a pump base unit (20).

15. The anal irrigation system according to claim 14, wherein the liquid container (30) is detachable from the pump base unit (20).

16. The anal irrigation system to claim 14 and 15, wherein the irrigation system further comprises tubes and an anal probe.

## Patentansprüche

1. Flüssigkeitsbehälter (30) für ein System zur analen Irrigation, wobei der Flüssigkeitsbehälter (30) einen oberen Teil (33) und eine Bodenwand umfasst, wobei mindestens eine Seitenwand (34) die Bodenwand (32) mit dem oberen Teil (33) verbindet und dabei einen Hohlraum zur Aufbewahrung von Flüssigkeit bildet, wobei der Flüssigkeitsbehälter (30) einen Einfüllstutzen (41) umfasst, der sich derart in der mindestens einen Seitenwand (34) befindet, dass der Einfüllstutzen (41) näher am oberen Teil (33) als am Bodenteil liegt, wobei der Einfüllstutzen (41) einen Umfang hat und wobei der Flüssigkeitsbehälter (30) ferner einen Griff (44) umfasst, **dadurch gekennzeichnet, dass** der Griff (44) um den Umfang des Einfüllstutzens (41) drehbar ist, wobei der Flüssigkeitsbehälter (30) dazu ausgelegt ist, dem Benutzer zu ermöglichen, den Behälter (30) in verschiedene Positionen (44a, 44b) anzuheben und zu halten.

2. Flüssigkeitsbehälter (30) nach Anspruch 1, wobei der Griff (44) in eine tangentiale Richtung rund um den Umfang und in einem Abstand von einem Zentrum des Einfüllstutzens drehbar ist.

3. Flüssigkeitsbehälter nach einem der vorhergehenden Ansprüche, wobei der Griff (44) mindestens eine erste und eine zweite Position (44a, 44b) bezogen auf den Flüssigkeitsbehälter (30) umfasst.

4. Flüssigkeitsbehälter nach Anspruch 3, wobei sich mindestens ein Teil des Griffs (44) über den oberen Teil (33) des Flüssigkeitsbehälters hinaus erstreckt, wenn der Griff (44) in der zweiten Position (44b) ist.

5. Flüssigkeitsbehälter nach einem der vorhergehenden Ansprüche, wobei der Griff (44) eine achtförmige Innenfläche mit zwei sich nach innen erstreckenden Flanschen (49) umfasst.

6. Flüssigkeitsbehälter nach einem der vorhergehenden Ansprüche, wobei der Griff (44) zwei Teile, einen Greifteil und einen Stutzenumfangsteil umfasst, die sich in zwei Ebenen erstrecken, die in Bezug zueinander winklig sind, wobei der Winkel zwischen den zwei Ebenen im Bereich von 0-45 Grad, z. B. 5-40 Grad, z. B. 10-30 Grad liegt.

7. Flüssigkeitsbehälter nach einem der vorhergehenden Ansprüche, wobei der Einfüllstutzen (41) in einer ersten Richtung (x) im Schwerpunkt des Flüssigkeitsbehälters (30) positioniert ist und in einer zweiten Richtung (y) vom Schwerpunkt des Flüssigkeitsbehälters (30) versetzt ist, wobei sich die erste Richtung (x) und die zweite Richtung (y) quer zueinander erstrecken.

8. Flüssigkeitsbehälter nach einem der vorhergehenden Ansprüche, wobei die Bodenwand (32) einen Auslassverbinder (31) zum fluidischen Verbinden des Flüssigkeitsbehälters (30) mit einer Pumpenbasiseinheit (20) umfasst.

9. Flüssigkeitsbehälter nach einem der vorhergehenden Ansprüche, wobei der Einfüllstutzen (41) derart in der mindestens einen Seitenwand (34) positioniert ist, dass der Einfüllstutzen (41) symmetrisch entlang der Mittellinie der mindestens einen Seitenwand (34) platziert ist.

10. Flüssigkeitsbehälter nach einem der vorhergehenden Ansprüche, wobei die Bodenwand (32) eine längliche gerundete rechtwinklige Form umfasst und der Einfüllstutzen (41) derart positioniert ist, dass er vertikal auf eine Mitte von einer der Seiten der rechtwinkligen Bodenwand (32) ausgerichtet ist.

11. Flüssigkeitsbehälter nach einem der vorhergehenden Ansprüche, wobei der Einfüllstutzen (41) einen starren ringförmigen Kragen umfasst, der den Umfang umgibt, wobei der Griff (44) drehbar mit dem ringförmigen Kragen verbunden ist.

12. Flüssigkeitsbehälter nach einem der vorhergehenden Ansprüche, wobei das Material des Griffs (44) aus starrem Kunststoffmaterial besteht.

13. Flüssigkeitsbehälter nach einem der vorhergehenden Ansprüche, wobei der Griff (44) eine Dicke von mehr als 2 mm umfasst.

14. System zur analen Irrigation, umfassend einen Flüssigkeitsbehälter (30) nach den vorhergehenden Ansprüchen und eine Pumpenbasiseinheit (20).

15. System zur analen Irrigation nach Anspruch 14, wobei der Flüssigkeitsbehälter (30) von der Pumpenbasiseinheit (20) lösbar ist.

16. System zur analen Irrigation nach Anspruch 14 und 15, wobei das System zur Irrigation ferner Schläuche und eine Analsonde umfasst.

## Revendications

1. Récipient de liquide (30) pour un système d'irrigation anale, le récipient de liquide (30) comprenant une partie supérieure (33) et une paroi inférieure, au moins une paroi latérale (34) reliant la paroi inférieure (32) avec la partie supérieure (33) formant une cavité de stockage de liquide, le récipient de liquide (30) comprenant un bec de remplissage (41) situé dans l'au moins une paroi latérale (34) de telle sorte que le bec de remplissage (41) soit plus près de la partie supérieure (33) que la partie inférieure, le bec de remplissage (41) ayant une périphérie, et le récipient de liquide (30) comprenant en outre une poignée (44), **caractérisée en ce que** la poignée (44) peut tourner autour de la périphérie du bec de remplissage (41), le récipient de liquide (30) étant configuré pour permettre à l'utilisateur de soulever et de maintenir le récipient (30) dans différentes positions (44a, 44b).

2. Récipient de liquide (30) selon la revendication 1, dans lequel la poignée (44) peut tourner dans une direction tangentielle autour de la périphérie et à une certaine distance du centre du bec de remplissage.

3. Récipient de liquide selon l'une quelconque des revendications précédentes, dans lequel la poignée (44) comprend au moins une première et une seconde position (44a, 44b) par rapport au récipient de liquide (30).

4. Récipient de liquide selon la revendication 3, dans lequel au moins une partie de la poignée (44) s'étend au-delà de la partie supérieure (33) du récipient de liquide, lorsque la poignée (44) se trouve dans la seconde position (44b).

5. Récipient de liquide selon l'une quelconque des revendications précédentes, dans lequel la poignée (44) comprend une face interne en forme de huit ayant deux brides s'étendant vers l'intérieur (49).

6. Récipient de liquide selon l'une quelconque des revendications précédentes, dans lequel la poignée (44) comprend deux parties, une partie de préhension et une partie de périphérie de bec, qui s'étendent dans deux plans inclinés l'un par rapport à l'autre, l'angle entre les deux plans étant dans la plage de 0 à 45 degrés, tel que 5 à 40 degrés, tel que 10 à 30 degrés.

7. Récipient de liquide selon l'une quelconque des revendications précédentes, dans lequel le bec de remplissage (41) est positionné au centre de gravité du récipient de liquide (30) dans une première direction (x) et décale le centre de gravité du récipient de liquide (30) dans une seconde direction (y), la première direction (x) et la seconde direction (y) s'étendant transversalement l'une à l'autre.

8. Récipient de liquide selon l'une quelconque des revendications précédentes, dans lequel la paroi inférieure (32) comprend un raccord de sortie (31) pour raccorder fluidiquement le récipient de liquide (30) à une unité de base de pompe (20).

9. Récipient pour liquide selon l'une quelconque des revendications précédentes, dans lequel le bec de remplissage (41) est positionné dans l'au moins une paroi latérale (34) de telle sorte que le bec de remplissage (41) soit placé symétriquement le long de l'axe central de l'au moins une paroi latérale (34).

10. Récipient de liquide selon l'une quelconque des revendications précédentes, dans lequel la paroi inférieure (32) comprend une forme rectangulaire arrondie oblongue et le bec de remplissage (41) est positionné de telle sorte qu'il soit aligné verticalement sur le milieu d'un des côtés de la paroi inférieure rectangulaire (32).

11. Récipient de liquide selon l'une quelconque des revendications précédentes, dans lequel le bec de remplissage (41) comprend un collier en forme d'anneau rigide entourant la périphérie, la poignée (44) étant reliée de manière rotative au collier en forme d'anneau.

12. Récipient de liquide selon l'une quelconque des revendications précédentes, dans lequel le matériau de la poignée (44) est réalisé en matière plastique rigide.

13. Récipient de liquide selon l'une quelconque des revendications précédentes, dans lequel la poignée (44) comprend une épaisseur de plus de 2 mm.

14. Système d'irrigation anale comprenant un récipient de liquide (30) selon les revendications précédentes et une unité de base de pompe (20).

15. Système d'irrigation anale selon la revendication 14, dans lequel le récipient de liquide (30) est détachable de l'unité de base de pompe (20).

16. Système d'irrigation anale selon les revendications 14 et 15, le système d'irrigation comprenant en outre des tubes et une sonde anale.
